# EUROPÄISCHE PATENTANMELDUNG

(11) **EP 2 725 563 A1**
(43) Veröffentlichungstag der Anmeldung: **30.04.2014**
(21) Anmeldenummer: 12007410.9
(22) Anmeldetag: 29.10.2012
(51) Int. Cl.: G08B 13/19, G08B 21/04, G08B 25/08, G08B 25/10, H04M 11/04

(54) **Sicherheitssystem mit integrierter Notruf-Funktion**

(71) Anmelder: Schori, Markus, 3052 Zollikofen (CH)
(72) Erfinder: Schori, Markus, 3052 Zollikofen (CH)
(74) Vertreter: Reb, Carina

(57) **Zusammenfassung**

Die Erfindung betrifft ein Sicherheitssystem (100) zur Bewegungserkennung (BE) in mindestens einem Raum, wobei die Bewegung mittels mindestens eines Distanzsensors (2) messbar ist und wobei in einem Mikrocontroller (15) ein Distanz-Erst-Istwert des Distanzsensors (2) mit einem Distanz-Zweit-Istwert des Distanzsensors (2) abgleichbar ist und ein Distanzmessungs-Signal (DmS) erzeugbar ist, mit dem mindestens ein Timer (16) triggerbar ist und nach Ablauf eines Zeitintervalls (ZI) ein systeminternes sensorseitiges Alarmsignal (ssA) ausgebbar ist.

## Beschreibung

Die vorliegende Erfindung betrifft ein Sicherheitssystem mit integrierter Notruf-Funktion für eine Wohnung, ein Ein- oder Mehrfamilienhaus, oder aber auch für soziale Einrichtungen wie Heime oder Spitäler.

Solche Sicherheitssysteme mit Notruf-Funktion basieren meist auf einem Infrarot-Bewegungsmelder und einem Timer und werden beispielsweise eingesetzt, um älteren Menschen noch so lange wie möglich selbständiges Wohnen zu ermöglichen, ihnen jedoch im Falle eines Notfalles Hilfe zukommen zu lassen. Hierbei löst das Bewegungsmelder-Signal das Ablaufen eines voreingestellten Zeitintervalls in dem Timer aus, und wenn während dieses Zeitintervalls kein weiteres Bewegungsmelder-Signal hinzukommt, sprich eine zu lange Inaktivität des Bewohners stattfindet, dann löst das Verstreichen des in dem Timer voreingestellten Zeitintervalls das Absetzen eines Notrufes aus.

Um sicher zu funktionieren, müssen solche Sicherheitssysteme viele Voraussetzungen erfüllen und beispielsweise hinsichtlich der Bewegungserfassung möglichst exakt zwischen der Bewegung eines Menschen und derjenigen eines Haustieres oder beispielsweise einem Luftzug in einer Gardine unterscheiden. Grundsätzlich jedoch sind die verwendeten Bewegungsmelder nicht in der Lage, eine qualitative Unterscheidung der Bewegungsart zu leisten.

Ein weiterer Nachteil bekannter Sicherheitssysteme ist, dass sie relativ unwirtschaftlich sind, weil sie ständig mit der erforderlichen Betriebsspannung gespeist sein müssen.

Die Aufgabe der vorliegenden Erfindung ist, unter Vermeidung der oben aufgezeigten Nachteile ein Sicherheitssystem mit integrierter Notruf-Funktion zu stellen, bei dem die Erfassung einer Bewegung und die Energieversorgung optimiert sind. Ausserdem soll das Sicherheitssystem über die Notruf-Funktion hinaus zahlreiche weitere Funktionen umfassen.

Die Lösung der Aufgabe besteht zunächst in der Auswahl eines Bewegungserfassungs-Systems, das vorzugsweise mikroprozessorunterstützt ist und mittels mindestens eines Distanzsensors eine Distanzmessung vornimmt. Dieses System ist erfindungsgemäss in der Lage, eine Veränderung eines vorgegebenen Distanz-Sollwertes zu einem neuen Distanz-Istwert oder die Veränderung eines Distanz-Erst-Istwertes zu einem neuen Distanz-Zweit-Istwert zu erfassen und dadurch ein gerätinternes (Delta eines) Distanzmessungs-Signal(s) abzusetzen.

Gemäss einer einfachsten Basis-Ausgestaltungsvariante eines erfindungsgemässen Sicherheitssystems, das im Wesentlichen lediglich den Distanzsensor und einen Timer umfasst, wird das Distanzmessungs-Signal des Distanzsensors dazu verwendet, den Timer einzuschalten.

Das Distanzmessungs-Signal kann jedoch vorzugsweise auch an einen in das Gerät integrierten Mikroprozessor bzw. Mikrocontroller weitergeleitet werden, in dem weiterhin erfindungsgemäss und gemäss einer bevorzugten Ausgestaltungsvariante ein Schwellenwert eingebbar ist. Dieser Schwellenwert ist vorzugsweise so eingestellt, dass erst dann, wenn der absolute Wert des Distanzmessungs-Signals ihn überschreitet, das Trigger-Signal an den Timer durch den Mikroprozessor ausgegeben wird.

Die Ermittlung des Schwellenwertes kann beispielsweise empirisch erfolgen und ist vorzugsweise auf allfällige spezielle örtliche Voraussetzungen adaptier- bzw. korrigierbar. Im Endeffekt jedenfalls besteht erfindungsgemäss eine durch den Mikroprozessor in Echtzeit erfolgende Unterscheidung zwischen geringfügigen, vernachlässigbaren Distanzmessungs-Signalen (Bewegung von Gegenständen, Haustieren etc.) und solchen, die zuverlässig auslösen sollen, weil beispielsweise eine Person durch den oder die Distanzsensoren erfasst worden ist.

Ein erfindungsgemässes Sicherheitssystem kann für die Distanzmessung einen oder mehrere Distanzsensoren umfassen, wobei alle gängigen Typen von Distanzsensoren in Betracht kommen. Es können licht- bzw. laserbasierte Signale ausgesandt werden, aber auch Infrarot- oder Terahertzstrahlung, Radio-, Mikrowellen oder Ultraschall. Kapazitive oder induktive Distanzmessungen kommen grundsätzlich auch in Betracht, auch wenn es schwieriger sein könnte, hierfür die erforderliche Spannungs- oder Energieversorgung vorzusehen. Eine weitere Möglichkeit der Distanzmessung kann mittels einer oder mehrerer elektronischen Kameras bzw. eines oder mehrerer CCDs erfolgen, die bzw. das die Parallaxen-Verschiebung bzw. die unterschiedlichen Stereobilder erfasst.

Wie bereits erwähnt, setzt vorzugsweise der Mikroprozessor dann, wenn der absolute Wert des Distanzmessungs-Signals den vorgegebenen Schwellenwert im Mikroprozessor übersteigt, ein Trigger-Signal ab, um den Timer einzuschalten. Vorzugsweise mittels des gleichen Mikroprozessors ist in diesem Timer ein bestimmtes Zeitintervall einstellbar, das nach dem Eingang des Distanzmessungs-Signals abzulaufen beginnt. Wenn das eingestellte Zeitintervall komplett abläuft, also die vermutete Inaktivität in dem betreffenden Raum verdächtig lange ist, dann setzt der Mikroprozessor ein sensorseitiges Alarmsignal ab. Wenn ein erneutes Distanzmessungs-Signal während dem begonnenen Ablauf des eingestellten Zeitintervalls eingeht, dann wird dieser Ablauf unterbrochen und es beginnt der Ablauf eines vollen neuen.

Das einstellbare Zeitintervall muss nicht fix sein, bevorzugt ist eine algorithmenbasierte dynamische Anpassung desselben, beispielsweise an Tag und Nacht, sodass z.B. nachts automatisch längere Zeitintervalle laufen.

Gemäss einer weiteren, bevorzugten Ausgestaltungsvariante eines erfindungsgemässen Sicherheitssystems ist dem mindestens einen Distanzsensor mindestens ein Bewegungsmelder vorgeschaltet. Mindestens der oder die Distanzsensoren und der oder die Bewegungsmelder sind vorzugsweise in einem gemeinsamen Gehäuse eines Gerätes untergebracht, das im Folgenden Sensorik-Gerät bezeichnet wird. Alle weiteren Module, der Mikrocontroller, das Notruf-Telefon, die Stromversorgung und sonstige Teile können grundsätzlich ebenfalls in dem Gehäuse des Sensorik-Gerätes angeordnet sein, oder aber auch in einem oder jeweiligen separaten, externen Gehäuse. Die letztere Anordnung kommt insbesondere dann in Betracht, wenn das Sicherheitssystem für ein ganzes Gebäude mit einer Vielzahl von Räumen oder Wohnungen ausgelegt sein soll und die Daten aller Sensorik-Geräte zentral gesammelt werden.

Der Bewegungsmelder kann grundsätzlich eine simple Lichtschranke sein oder aber beispielsweise ein passiver Infrarot-Sensor. Es kommen aber auch ultraschallbasierte Bewegungsmelder in Betracht, oder solche, die mit elektromagnetischen Wellen funktionieren (Radare, Dopplerradare oder auch sogenannte Bio-Radare, die darauf ausgelegt sind, auch kleinste Bewegungen einer Person zu erfassen, beispielsweise die Atmungsbewegungen oder den Herz- oder/und Pulsschlag einer verschütteten Person).

Der Bewegungsmelder benutzt erfindungsgemäss die Erfassung einer beliebigen Bewegung in dem Raum, in welchem er angeordnet ist, aber vorzugsweise lediglich nur, um ein Auslösesignal an den Mikroprozessor bzw. den Distanzsensor auszugeben. Eine qualitative Erfassung der Art der Bewegung durch den Bewegungsmelder unterbleibt und erfolgt erst durch die Distanzmessung. Dieser Aufbau ist bevorzugt, es wäre aber auch möglich, beide Signale (Distanz und Bewegung) auszuwerten.

Um trotzdem bereits mittels des Bewegungsmelders eine zuverlässige Sensitivität, aber auch eine gewisse Selektivität zu erreichen, ist es bevorzugt, ein erfindungsgemässes Sicherheitssystem mit einem Bewegungsmelder auszustatten, der von seiner geometrischen Definition her und allfälligen optischen Elementen vorzugsweise in approximativer Schalterhöhe in einem Bereich von 0.5 bis 1.5 m, vorzugsweise ca. 1.1 m angeordnet ist und weiterhin vorzugsweise jeweils einen Öffnungswinkel in einem Bereich von 5 bis 90 Grad, vorzugsweise ca. 30 Grad sowohl für einen horizontalen, als auch für einen vertikalen Erfassungsbereich aufweist. Die Nennreichweite für den Bewegungsmelder liegt vorzugsweise in einem Bereich von 0.5 bis 10 m und beträgt vorzugsweise 2.0 m. Die Nennreichweite des Distanzsensors kann geringer sein und liegt vorzugsweise in einem Bereich von 0.5 bis 8 m und beträgt vorzugsweise 1.3 m.

Davon ausgehend, dass die durchschnittliche Geschwindigkeit eines Fussgängers zwischen ca. 1.8 km/h (= 0.5 m/s; bei älteren Menschen oder Gehbehinderten) und ca. 6.5 km/h (= 1.8 m/s) betragen kann, liegt die bevorzugte Geschwindigkeitserfassung, in denen der Bewegungsmelder hinsichtlich seiner Leistungsfähigkeit vorzugsweise anspricht, in einem Bereich von 0.1 bis 3 m/s und beträgt vorzugsweise 0.5 m/s. Auf diese Weise ist eine sichere Erfassung einer Person gewährleistet, ein schneller vorbeilaufendes oder -springendes Haustier oder gar eine vorbeifliegende Fliege wird jedoch nicht erfasst.

Die Stromversorgung des Distanzsensors - und vorzugsweise auch des Mikroprozessors - ist vorzugsweise an das Auslösesignal des Bewegungsmelders gekoppelt, d.h., dass der Distanzsensor - und vorzugsweise auch der Mikroprozessor - nicht ständig mit Strom versorgt ist, sondern nur dann, wenn er durch das Auslösesignal des Bewegungsmelders in Funktion gesetzt wird. In diesem Zusammenhang ist es konsequent, einen möglichst stromsparenden Bewegungsmelder auszusuchen und mit dessen Hilfe lediglich ein das Sicherheitssystem einschaltendes bzw. "scharf" machendes Detektionssignal zu verwenden.

Zusätzlich dazu ist es bevorzugt, für den Bewegungsmelder einen Energiesparmodus vorzusehen, aus dem er dann in einen Normalspannungsmodus wechselt, wenn eine Bewegung registriert wird oder die Zeit des Intervalls überschritten wird.

Es ist des Weiteren bevorzugt, dass mittels des Mikroprozessors oder/und eines Dreh-, Dreh-Schiebe-Schalters oder DIP-Schalters unterschiedliche Zeitintervalle manuell einstellbar sind, zusätzlich zu einer weiterhin vorzugsweise angeordneten Ethernet- oder KNX-Schnittstelle. Weiterhin vorzugsweise sind fix voreingestellte Zeitintervalle auswählbar, je nach dem Raum, in dem die Distanzmessung erfolgen soll, beispielsweise ein Zeitintervall Schlafzimmer, ein Zeitintervall Wohnzimmer oder ein Zeitintervall Badezimmer. Des Weiteren ist es vorgesehen, dass die Einstellmöglichkeit des Zeitintervalls mechanisch oder/und elektronisch gegen zufälliges Verstellen gesichert ist.

Optional kann die Einstellung des Zeitintervalls oder die Bedienung aller sonstigen Funktionen eines erfindungsgemässen Sicherheitssystems auch mit einer Fernbedienung erfolgen, wobei beispielsweise Infrarot- oder Funkfernbedienungen in Betracht kommen, mit entsprechenden Empfängern an einem Gerät, das mindestens den Mikrocontroller umfasst. Die Funktion einer Fernbedienung können auch Smartphones, Laptops, PCs und Tablets übernehmen, sofern eine Ethernet-Schnittstelle oder ein Modem oder ein Funksender vorgesehen ist.

Weiterhin vorzugsweise umfasst ein erfindungsgemässes Sicherheitssystem ein Kommunikationsmodul für systemexterne Kommunikation. Dieses kann ein bidirektionales GSM-, KNX- oder TCPIP-basiertes Interface sein oder zwei oder alle drei Kommunikationsarten umfassen. Die Einstellung des Zeitintervalls, aber auch die Auslösung und Überwachung sämtlicher Funktionen des Sicherheitssystems können auf diese Weise auch von extern erfolgen.

Für sämtliche Konfigurationsmodi eines erfindungsgemässen Sicherheitssystems ist es mittels einer Timeout-Schaltung vorgesehen, dass es bei zu langem Konfigurationsbetrieb oder bei fehlenden Bestätigungseingaben automatisch in die ursprüngliche Konfiguration und den Normalbetrieb zurückzufallen, welcher der ursprünglichen Konfiguration entspricht.

Bei einem erfindungsgemässen Sicherheitssystem sind vorzugsweise entsprechende Schalter und eine entsprechende Software für das Umschalten des Sicherheitssystems in einen Abwesenheits- bzw. Ferienmodus vorgesehen. In diesem Modus sind der oder die Distanzsensoren oder der oder die Bewegungsmelder oder alle Sensoren deaktiviert und vorzugsweise auch der oder die Timer. Um das Sicherheitssystem nach Rückkehr aus der Abwesenheit oder den Ferien wieder in den Normalbetrieb zu aktivieren, können einerseits Anzeigen vorgesehen sein, die vorzugsweise sowohl akustisch, als auch optisch zyklisch anzeigen, dass das Sicherheitssystem im Abwesenheits- bzw. Ferienmodus ist. Dadurch wird die zurückgekehrte Person darauf hingewiesen, dass sie das Sicherheitssystem wieder einschalten muss.

Andererseits kann es vorgesehen sein, mindestens den oder die Bewegungsmelder mit einer Ruhemodus-Spannung zu speisen und so das Sicherheitssystem automatisch bei einer Bewegungserfassung aus dem Abwesenheits- bzw. Ferienmodus wieder in den Normalbetrieb "hochzufahren". Für eine Ausgestaltungsvariante eines erfindungsgemässen Sicherheitssystems mit so einer automatischen Rückschaltung aus dem Abwesenheits- bzw. Ferienmodus ist es vorgesehen, dass die akustischen und optischen Anzeigen für eine bestimmte Zeitdauer ein quittierbares Warnsignal abgeben. Auf diese Weise ist gewährleistet, dass eine Reinigungskraft oder ein die Blumen giessender Nachbar darauf hingewiesen wird, das Sicherheitssystem nach Erledigung seiner Arbeit wieder in den Abwesenheits- bzw. Ferienmodus zu schalten.

Die Umschaltung in den Abwesenheits- bzw. Ferienmodus erfolgt vorzugsweise mit einer timerunterstützten Zeitverzögerung, damit eine Bewegung der sich entfernenden Person nicht bereits als ein Rückschaltungssignal in den Normalmodus interpretiert wird.

Nicht nur das oben beschriebene Warnsignal ist vorzugsweise mittels eines Bestätigungsschalters quittierbar, sondern auch alle anderen Alarmsignale des Sicherheitssystems, sowohl das sensorseitige Alarmsignal, als auch das telefonseitige, sprich dem tatsächlichen Absetzen des Notrufs. Diese Funktion ist für das Abstellen allfälliger Fehlalarme vorgesehen, aber auch für das Abstellen des Alarms, nachdem das Rettungspersonal eingetroffen ist.

Des Weiteren sind bei einem erfindungsgemässen Sicherheitssystem Schalter und entsprechende Software vorgesehen, um einen Testalarm auszulösen, sowohl für den externen telefonseitigen Alarm, als auch für den internen sensorseitigen. Die letztere Testfunktion ist vorzugsweise mit einer Zeitraffer-Schaltung des Timers kombinierbar, sodass nicht das Verstreichen des gesamten eingestellten Inaktivitäts-Zeitintervalls abgewartet werden muss.

Ein erfindungsgemässes Sicherheitssystem führt, ebenfalls timerunterstützt, automatisch und zyklisch einen Selbsttest im Normalbetrieb durch, von dem Bewegungsmelder, dem Distanzsensor, dem Timer, der Software, bis über das Notruf-Telefon. Die Selbsttest-Funktion ist vorzugsweise so eingerichtet, dass die Daten eines fehlerhaften Selbsttests oder mindestens die Tatsache, dass ein Selbsttest fehlerhaft verlief, als Fehlerprotokoll in einem Fehlerspeicher ablegbar ist. Dieser Fehlerspeicher ist weiterhin vorzugsweise mittels eines externen Diagnosegeräts auslesbar, das an einem Diagnoseanschluss anschliessbar ist, der wiederum vorzugsweise an demjenigen Gehäuse angeordnet ist, in dem der Mikrocontroller untergebracht ist. Des Weiteren wird der Zustand des Fehlerspeichers vorzugsweise am Gehäuse des Sicherheitssystems bzw. des Mikrocontrollers optisch oder akustisch angezeigt.

Der abgespeicherte Fehler dient weiterhin erfindungsgemäss auch dafür, bei einem darauffolgenden, erneuten fehlerhaften Selbsttest einen Notruf abzusetzen, vorzugsweise mit einer Kennung, dass es sich um einen Technik-Notfall und keinem Personen-Notfall handelt. Wenn der darauffolgende oder aus Gründen der Redundanz mehrere der folgenden Selbsttests fehlerfrei verlaufen sind, dann kann es optional vorgesehen sein, dass der abgespeicherte Fehler automatisch gelöscht wird.

Die internen Schaltungen und die Software sind vorzugsweise so konfiguriert bzw. separiert, dass die automatischen Selbsttests keine Unterbrechung des Normalbetriebs verursachen können. Es ist lediglich eine optische oder akustische Anzeige vorgesehen, welche die gerade stattfindende Durchführung des Selbsttests anzeigt. Des Weiteren ist es vorgesehen, dass nach jedem Neustart automatisch ein Selbsttest startet, beispielsweise bei Inbetriebnahme und nach einem Batterie- oder Akkuwechsel oder Stromunterbruch.

Wie bereits erwähnt, umfasst ein erfindungsgemässes Sicherheitssystem ein Notruf-Telefon oder eine integrierte Telefon-Einheit, die so geschaltet ist, dass der sensorseitige Alarm ein Wählen ringgespeicherter Notrufnummern auslöst. Das Auslösen des Notruf-Telefons erfolgt erfindungsgemäss mittels eines systeminternen Kommunikationsmoduls. Hierfür kann eine herkömmliche Telefonleitung angeordnet sein, oder ein funkbasiertes Sendemodul, beispielsweise ein ELDAT-Kommunikationsmodul, oder aber auch ein GSM-, KNX- oder Ethernet-Kommunikationsmodul oder eine Kombination eines Teils dieser Kommunikationsmodule oder eine Kombination aller. Ein solches systeminternes Kommunikationsmodul muss nicht zwingend bidirektional sein, es genügt die unidirektionale Kommunikation mittels einer sogenannten Simplex-Verbindung des sensorseitigen Alarms an das Notruf-Telefon, allerdings ist es bevorzugt, auch diese bidirektional in Form einer sogenannten Duplex-Verbindung zu realisieren, sodass bei bereits ausgelöstem Notruf-Telefon ein sogenanntes "Acknowledge" (ein positives Rückbestätigungs-Signal) des Notruf-Telefons die wiederholte Absetzung des sensorseitigen Alarms stoppt.

Die Telefon-Einheit ist vorzugsweise in der Lage, erfolglos z.B. auf einem Anrufbeantworter eingegangene Anrufe zu erkennen, beispielsweise indem ein Bestätigungssignal lesbar ist.

Zur Erhöhung der Übertragungssicherheit zwischen dem jeweiligen systeminternen Kommunikationsmodul und dem Notruf-Telefon kann im Falle einer Simplex-Verbindung eine TimerSchaltung angeordnet sein, die eine redundante Wiederholung des sensorseitigen Alarms an das Notruf-Telefon so lange in Gang setzt, bis eine Quittierung am Notruf-Telefon erfolgt.

Bei einem erfindungsgemässen Sicherheitssystem erfolgt die sogenannte Verheiratung zwischen Sensorik und Notruf-Telefon vorzugsweise mit einer spezifischen Identifikation kodiert. D.h., dass das jeweilige Kommunikationsmodul eine eigene, spezifische Frequenz und/oder Kennung sendet und das jeweilige Empfangsmodul an dem Notruf-Telefon diese zuerst einlernen muss, um logisch miteinander verbunden zu sein. Hierfür sind sowohl an den Kommunikationsmodulen der Sensorik, als auch an dem Notruf-Telefon Tasten angeordnet, um die Einlern-Funktion zu aktivieren. Auf diese Weise ist gesichert, dass Signale eines benachbarten Sicherheitssystems oder anderweitige Signale nicht zu einer ungewollten Auslösung eines telefonseitigen Notruf-Alarms führen.

Optional kann es bei einem erfindungsgemässen Sicherheitssystem vorgesehen sein, dass die jeweiligen Sende- bzw. Übertragungsmodule der Kommunikations-Schnittstellen die durch das jeweilige Empfangsmodul einzulernende Frequenz oder/und Kennung während der aktivierten Einlern-Funktion beispielsweise nur stossweise in Impulsen aussenden. Das jeweilige Empfangsmodul ist so programmiert, einerseits die Frequenz und/oder Kennung zu lernen, aber andererseits dieses ausschliesslich nur von der stossweise in Impulsen gesendeten, zu erlernenden Frequenz und/oder Kennung. Ansonsten wäre man bei der Installation eines neuen Sicherheitssystems gezwungen sicherzustellen, dass in Reichweite liegende Sicherheitssysteme abgeschaltet sind oder auch sonst keine anderweitigen Signale vorliegen, die von dem Empfangsmodul des Notruf-Telefons fälschlicherweise eingelernt werden könnten.

Ein erfindungsgemässes Sicherheitssystem kann am Stromnetz angeschlossen sein, weist jedoch vorzugsweise eine Stromversorgung mittels eines oder mehrerer Akkus oder mittels einer oder mehrerer Batterien auf. Weiterhin erfindungsgemäss ist in dem Sicherheitssystem eine Schaltung zum Beispiel mit einem Spannungsregler bzw. -detektor angeordnet, welche die Akku- oder Batteriespannung überwacht. In dieser Schaltung ist eine Mindestspannung als Schaltschwelle eingebbar, wobei bei einer Annäherung an den eingegebenen Mindestspannungs-Wert oder Unterschreitung desselben der oder die Timer zur Absetzung eines Alarms geschaltet werden. Dem Absetzen des Alarms ist vorzugsweise eine optische und später akustische Warnsignal-Gebung vorgeschaltet, die ca. 2-3 Tage vorher erfolgt. Des Weiteren ist vorzugsweise ein Schalter angeordnet, mittels dessen der Kunde die Möglichkeit hat, das Warnsignal einmalig um ca. 12 Stunden zu "stunden", beispielsweise für den Fall, dass die Spannungs-Unterschreitung nachts passiert.

Optional kann es vorgesehen sein, die Funktion der Spannungsüberprüfung ebenfalls an den Timer zu koppeln. Auf diese Weise können Spannungsschwankungen, welche die Schaltschwelle nur kurzzeitig unterschreiten, noch keinen Alarm auslösen, sondern nur solche, die sie über einen einstellbaren Zeitraum unterschreiten.

Sowohl der sensorseitige, quasi gerät- bzw. systeminterne Alarm, als auch der telefonseitige Notfall-Alarm, haben weiterhin erfindungsgemäss eine Voralarm-Vorstufe, die dem Kunden oder Servicepersonal eine Quittier- bzw. Interventionsmöglichkeit gibt. Hierfür sind an dem Gerät vorzugsweise für den jeweiligen Alarm optische und akustische Signalgeber angeordnet und jeweils eine entsprechende Direkttaste, die den jeweiligen Alarm unverzüglich stoppt. Diese Direkttaste kann mit einer mechanischen Sperre gesichert sein. Für die Quittier- bzw. Interventionsmöglichkeit ist vorzugsweise eine Schaltung zu dem Timer angeordnet, die den Ablauf eines Zeitintervalls startet, innerhalb dessen der Voralarm quittierbar ist. Wenn dieses Zeitintervall ohne Quittierung abläuft, dann wird der vollwertige sensor- oder telefonseitige Alarm geschaltet.

Ein erfindungsgemässes Sicherheitssystem umfasst vorzugsweise mindestens einen nichtflüchtigen Speicher für das Abspeichern der funktions- bzw. systemrelevanten Daten wie zum Beispiel dem eingestellten Zeitintervall, Konfigurationsdaten des Distanzsensors und des Bewegungsmelders, der Kommunikations-Schnittstellen, des Notruf-Telefons, der Alarmabfolgen usw.

Die Gehäuseteile eines erfindungsgemässen Sicherheitssystems sind vorzugsweise aus Kunststoff oder aber auch aus Metall ausgeformt und so ausgestaltet, dass sie der Schutzklasse IP 20 genügen, vorzugsweise jedoch mindestens der Schutzklasse IP 24 (spritzwassergeschützt in feuchten Räumen). Das Gehäuse kann eine Oben-Unten-Markierung aufweisen, sodass der Monteur einen Montagehinweis bekommt und ein allfälliges Batteriefach nicht oberhalb der Elektronik anordnet. Das Batteriefach bildet vorzugsweise eine Wanne aus, die das Austreten von Batterieflüssigkeit verhindert. Des Weiteren weist das Gehäuse vorzugsweise eine Fläche oder Kantenpaare aus, an welche bei der Montage zur exakten Ausrichtung der Sensoren eine Wasserwaage anlegbar ist. Des Weiteren ist das Gehäuse vorzugsweise so abgerundet aufgebaut, dass Dinge des täglichen Gebrauchs, wie z.B. Schlüssel, Portemonnaies, Kopfbedeckungen, Handschuhe oder Schals nicht oder nur sehr schwer darauf abgelegt werden können.

Die Gehäuseteile, aber auch die Schnittstellen und nicht zuletzt die Software, will heissen die gesamte Architektur eines erfindungsgemässen Sicherheitssystems ist vorzugsweise modular aufgebaut, sodass eine Basis-Variante nach Kundenwünschen konfigurier- oder erweiterbar ist und/oder Einzelkomponenten austauschbar sind.

Die Software eines erfindungsgemässen Sicherheitssystems verfügt vorzugsweise über eine timergesteuerte Update-Funktion.

Die systeminternen, aber insbesondere die systemexternen bidirektionalen Kommunikationsmodule sind vorzugsweise um Schnittstellen für beliebige weitere Funktionen eines erfindungsgemässen Sicherheitssystems erweiterbar. Hierbei kommt der Eingang und die Verarbeitung von unterschiedlichen Sensorsignalen in Betracht, wie z.B. einem Sensorsignal vom Türschloss der Eingangstüre (offen / geschlossen / abgesperrt), gebäudetechnischen Sensorsignalen (Licht / Heizung / Fenster / Storen / Elektro-Schalt- und Sicherungstafel), Sensorsignalen des Küchenherdes (an / aus / kein Gefäss auf eingeschalteter Herdplatte), Sensorsignal von einem Überlaufschutz der Badewanne, Sensorsignalen den Gesundheitszustand betreffend (Herzfrequenz / Puls / Blutdruck u.a.) sowie einem Sensorsignal einer Kamera oder eines Scanners, die optisch beispielsweise das Belegungsbild der Fächer einer Tablettenschachtel mit der vorbereiteten Medikation erfasst und mit einem gespeicherten Bild abgleicht.

Die vorliegende Anmeldung offenbart ein Verfahren zur Erfassung der Inaktivität in einem oder mehreren Räumen und einem Absetzen eines Notrufes nach Verstreichen eines voreingestellten Zeitintervalls mit einem wie offenbarten Sicherheitssystem, mit folgenden grundsätzlichen Verfahrensschritten:
a) - Erfassen einer Bewegung mittels eines Bewegungsmelders;
b) - Einschalten eines Distanzsensors bei einer erfassten Bewegung;
c) - Durchführen einer Distanzmessung zu dem bewegten Objekt oder bewegten Person und Weiterleiten eines Distanzmessungs-Signals an einen Mikroprozessor;
d) - Abgleichen des Distanzmessungs-Signals mit einem vorgegebenen Schwellenwert;
e) - Einschalten eines Timers bei Überschreiten des Schwellenwerts;
f) Absetzen eines sensorseitigen Alarms bei Verstreichen eines vorgegebenen Zeitintervalls des Timers;
g) - Auslösen eines telefonseitigen Alarms durch Einschalten eines Notruf-Telefons.

Eine erfindungsgemässes Sicherheitssystem bringt folgende Vorteile:
- Die Bewegungserfassung erfolgt nicht nur an und für sich bzw. quantitativ durch den Bewegungssensor, sondern qualitativselektiv durch den Distanzsensor.
- Die Stromversorgung ist optimiert.
- Es ist besser an örtliche und individuelle Gegebenheiten anpassbar.
- Es ist von extern überwach- und steuerbar.
- Es verfügt über einen Abwesenheits- bzw. Ferienmodus.
- Die Alarmsignale haben eine quittierbare Vorstufe.
- Es verfügt über eine Selbsttest-Funktion, die den Normalbetrieb nicht unterbricht.
- Die Installation und der Betrieb sind so optimiert, dass benachbarte Sicherheitssysteme oder Signale keine Berücksichtigung finden.
- Es verfügt über eine Spannungsüberwachung.
- Es ist modular aufgebaut und somit gut erweiter- oder reparierbar.
- Es ist für den Eingang und die Verarbeitung von vielen weiteren Sensorsignalen ausgelegt, wie z.B. gebäude-, sicherheitstechnischen und individuell-gesundheitlichen Signalen.

Weitere oder vorteilhafte Ausgestaltungen eines erfindungsgemässen Sicherheitssystems bilden die Gegenstände der abhängigen Ansprüche.

Die Bezugszeichenliste ist Bestandteil der Offenbarung.

Anhand von Figuren wird die Erfindung symbolisch und beispielhaft näher erläutert. Die Figuren werden zusammenhängend und übergreifend beschrieben. Sie stellen schematische und beispielhafte Darstellungen dar und sind nicht massstabsgetreu, auch in der Relation der einzelnen Bestandteile zueinander nicht. Gleiche Bezugszeichen bedeuten das gleiche Bauteil, Bezugszeichen mit unterschiedlichen Indices geben funktionsgleiche oder ähnliche Bauteile an.

Es zeigen dabei
Fig. 1 eine schematische Darstellung einer beispielhaften Ausgestaltungsvariante eines erfindungsgemässen Sicherheitssystems;
Fig. 2 eine schematische Darstellung in Seitenansicht einer beispielhaften Anordnung der Sensorik eines erfindungsgemässen Sicherheitssystems und
Fig. 3 eine schematische Darstellung in Draufsicht der beispielhaften Anordnung der Sensorik des erfindungsgemässen Sicherheitssystems aus der Fig. 2.

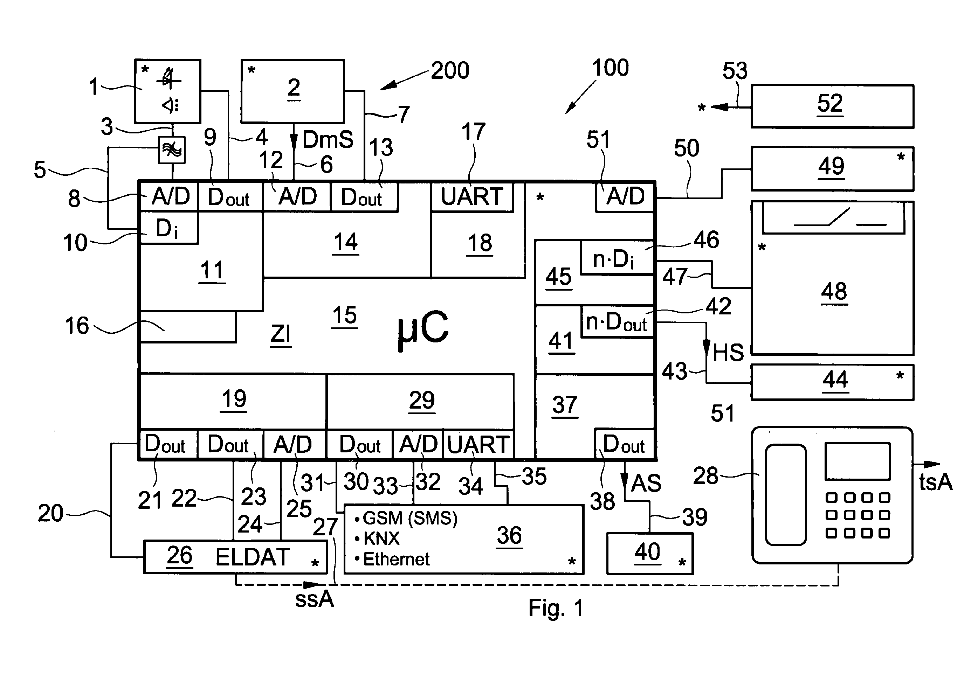

In der Fig. 1 ist eine beispielhafte Ausgestaltungsvariante eines erfindungsgemässen Sicherheitssystems 100 schematisch dargestellt. Das Sicherheitssystem 100 umfasst einen Infrarot-Bewegungsmelder 1 und einen Distanzsensor 2, die zusammen eine Sensorik 200 bilden. Der Infrarot-Bewegungsmelder 1 ist mit einer Verbindungsleitung 3 zu einem Analog-Digital-Anschluss 8 mit einem Bewegungsmelder-Interface 11 verbunden, das des Weiteren einen digitalen Ausgang 9 und einen digitalen Eingang 10 umfasst. An dem digitalen Ausgang 9 ist eine Testleitung 4 für Tests des Infrarot-Bewegungsmelders 1 angeschlossen. An dem digitalen Eingang 10 ist eine Interruptsignal-Leitung 5 angeordnet.

Der Distanzsensor 2 ist an ein Distanzsensor-Interface 14 angeschlossen, mittels einer Verbindungsleitung 6 zu einem Analog-Digital-Anschluss 12 und einer Einschaltleitung 7 von einem digitalen Ausgang 13. Das Bewegungsmelder-Interface 11 und das Distanzsensor-Interface 14 sind an einem Mikrocontroller 15 angeordnet. Durch die beschriebenen Anschlüsse und Komponenten ist gewährleistet, dass der Distanzsensor 2 erst nach einem Signal des Infrarot-Bewegungsmelders 1 einschaltbar und wenn dieses der Fall ist, der Infrarot-Bewegungsmelder 1 wieder ausschaltbar ist.

Das Sicherheitssystem 100 umfasst des Weiteren einen Timer 16 sowie ein Test-Interface 18 mit einer UART-Schnittstelle 17. Die dargestellte Ausgestaltungsvariante eines erfindungsgemässen Sicherheitssystems 100 umfasst weiterhin ein durch den Mikrocontroller 15 gesteuertes simples Kommunikations-Interface 19, das über eine an einem digitalen Ausgang 21 angeschlossene Einschaltleitung 20 einen Funksender 26 einschaltet. Dieser Funksender 26 ist auch mit einer permanenten Verbindungsleitung 22 an einem weiteren digitalen Ausgang 23 des Kommunikations-Interfaces 19 angeschlossen. Zu Testzwecken steht eine Testleitung 24 zur Verfügung, die an einem Analog-Digital-Anschluss 25 angeordnet ist.

Wenn von dem Distanzsensor 2 über die Verbindungsleitung 6 bzw. den Analog-Digital-Anschluss 12 des Distanzsensor-Interfaces 14 ein Distanzmessungs-Signal DmS an den Mikrocontroller 15 gelangt, so wird der Timer 16 getriggert bzw. zurückgesetzt. Falls ein in dem Mikrocontroller 15 hinterlegtes Zeitintervall ZI überschritten wird, so schaltet der Mikrocontroller 15 über die Einschaltleitung 20 den Funksender 26 ein und der wiederum sendet dann einen sensorseitigen Alarm ssA über eine gestrichelt dargestellte Funkverbindung 27 an eine Notruf-Telefoneinheit 28. Diese wiederum gibt einen telefonseitigen Alarm tsA an die in einem Ringspeicher hinterlegten Notrufnummern aus.

Die dargestellte Ausgestaltungsvariante eines erfindungsgemässen Sicherheitssystems 100 umfasst des Weiteren ein komplexes Kommunikations-Interface 29, das über einen digitalen Ausgang 30 und eine Einschaltleitung 31 ein Kommunikationsmodul 36 einschalten kann. Das dargestellte Kommunikationsmodul 36 beherrscht GSM-, KNX- und Ethernet-Kommunikation, es sind jedoch auch einzelne, separate Kommunikationsmodule für jede Übertragungsart möglich. Das Kommunikationsmodul 36 und das komplexe Kommunikations-Interface 29 sind des Weiteren über eine Testleitung 33 zu einem Analog-Digital-Anschluss 32 und eine permanente Verbindungsleitung 35 zu einer UART-Schnittstelle 34 verbunden.

Das Sicherheitssystem 100 umfasst des Weiteren eine Alarmausgabe-Schnittstelle 37, die ein Alarmsignal AS über einen digitalen Ausgang 38 und eine Verbindungsleitung 39 an eine Alarmeinheit 40 ausgibt. Die Alarmeinheit 40 kann sowohl akustische, als auch optische Alarmeinrichtungen umfassen.

Ein Hinweissignale-Interface 41 gibt Hinweissignale HS aus, wie beispielsweise den Hinweis betreffend, dass eine Detektion stattgefunden hat oder dass sich das Sicherheitssystem 100 im Abwesenheits- bzw. Ferienmodus befindet oder dass gerade ein Selbsttest stattfindet oder dass ein Selbsttest fehlgeschlagen ist oder dass sich das Sicherheitssystem gerade in einem Konfigurationsmodus befindet. Diese Hinweissignale HS werden über mindestens einen digitalen Ausgang 42 des Hinweissignale-Interfaces 41 und eine Verbindungsleitung 43 an eine Signalisationseinrichtung 44 wie z.B. ein Display oder eine Anordnung von Leuchten geleitet.

Die dargestellte Ausgestaltungsvariante eines erfindungsgemässen Sicherheitssystems 100 umfasst des Weiteren ein Benutzereingabe-Interface 45 mit mindestens einem digitalen Eingang 46 und einer Verbindungsleitung 47, in das Steuer- bzw. Schaltsignale SS durch einen Benutzer oder technisches Servicepersonal über eine Steuer- und Schalteinheit 48 eingebbar sind.

Eine Spannungsüberwachung 49 ist mittels einer Verbindungsleitung 50 an einem Analog-Digital-Anschluss 51 angeschlossen.

Eine Energieversorgung 52 versorgt alle Komponenten des Sicherheitssystems 100, die mit einem Sternchen gekennzeichnet sind. Somit sind die entsprechenden Stromleitungen lediglich symbolisch durch einen Stromausgang 53 dargestellt.

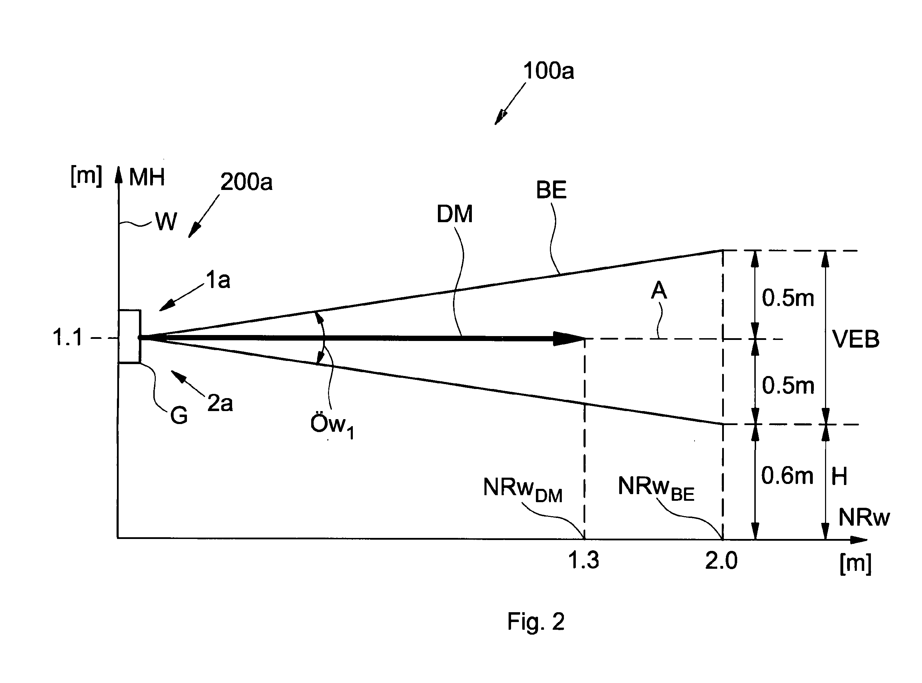

Die Fig. 2 zeigt ebenfalls schematisch und in Seitenansicht eine beispielhafte Anordnung einer Sensorik 200a eines erfindungsgemässen Sicherheitssystems 100a, die einen Bewegungsmelder 1a und einen Distanzsensor 2a in einem Gehäuse G umfasst. Die Sensorik 200a bzw. das annähernde Zentrum des Gehäuses G ist an einer Wand W vorzugsweise in einer Montagehöhe MH von 1.1 m angeordnet.

Eine Bewegungserkennung BE durch den Bewegungsmelder 1a erfolgt in einem Öffnungswinkel Öw₁ von ca. 30 Grad und ergibt somit in einer Bewegungserkennungs-Nennreichweite NRw_{BE} von ca. 2.0 m einen vertikalen Erfassungsbereich VEB von jeweils 0.5 m ober- und unterhalb einer Achse A in einer Höhe H von ca. 0.6 m. Eine Distanzmessung DM durch den Distanzsensor 2a erfolgt linear bis in eine Distanzmessungs-Nennreichweite NRw_{DM} von 1.3 m. Die Distanzmessung DM entspricht der Achse A, grundsätzlich jedoch kann die Distanzmessung DM auch in jede beliebige andere Richtung erfolgen.

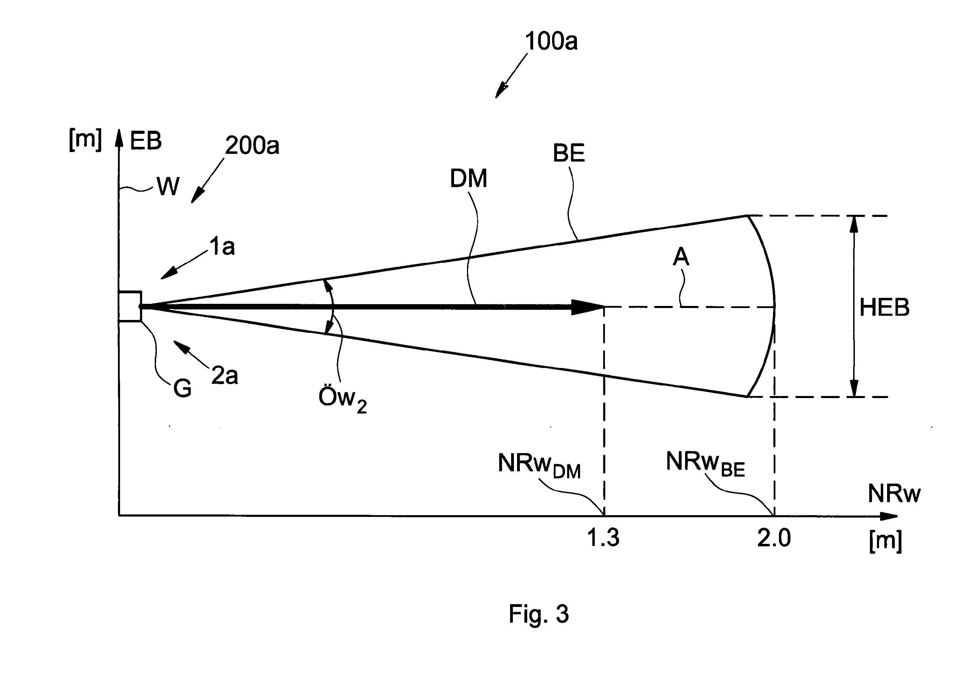

Die Fig. 3 zeigt schematisch die Draufsicht der beispielhaften Anordnung der Sensorik 200a des erfindungsgemässen Sicherheitssystems 100a aus der Fig. 2. Auf der Y-Achse ist nun eine Erfassungsbreite EB dargestellt, die bei einem ebenfalls ca. 30-grädigen Öffnungswinkel Öw₂ einen horizontalen Erfassungsbereich HEB der Bewegungserkennung BE bzw. des Bewegungsmelders 1a ergibt.

**Bezugszeichenliste**

| | |
|---|---|
| 1, 1a | - Infrarot-Bewegungsmelder, Bewegungsmelder |
| 2, 2a | - Distanzsensor |
| 3 | - Verbindungsleitung |
| 4 | - Testleitung |
| 5 | - Interruptsignal-Leitung |
| 6 | - Verbindungsleitung |
| 7 | - Einschaltleitung |
| 8 | - Analog-Digital-Anschluss |
| 9 | - digitaler Ausgang |
| 10 | - digitaler Eingang |
| 11 | - Bewegungsmelder-Interface |
| 12 | - Analog-Digital-Anschluss |
| 13 | - digitaler Ausgang |
| 14 | - Distanzsensor-Interface |
| 15 | - Mikrocontroller, Mikroprozessor |
| 16 | - Timer |
| 17 | - UART-Schnittstelle |
| 18 | - Test-Interface |
| 19 | - simples Kommunikations-Interface |
| 20 | - Einschaltleitung |
| 21 | - digitaler Ausgang |
| 22 | - permanente Verbindungsleitung |
| 23 | - digitaler Ausgang |
| 24 | - Testleitung |
| 25 | - Analog-Digital-Anschluss |
| 26 | - Funksender |
| 27 | - Funkverbindung |
| 28 | - Notruf-Telefoneinheit |
| 29 | - komplexes Kommunikations-Interface |
| 30 | - digitaler Ausgang |
| 31 | - Einschaltleitung |
| 32 | - Analog-Digital-Anschluss |
| 33 | - Testleitung |
| 34 | - UART-Schnittstelle |
| 35 | - permanente Verbindungsleitung |
| 36 | - Kommunikationsmodul |
| 37 | - Alarmausgabe-Schnittstelle |
| 38 | - digitaler Ausgang von 37 |
| 39 | - Verbindungsleitung |
| 40 | - Alarmeinheit |
| 41 | - Hinweissignale-Interface |
| 42 | - digitaler Ausgang von 41, wobei n ganze Zahl grösser Null |
| 43 | - Verbindungsleitung |
| 44 | - Signalisationseinrichtung, Display |
| 45 | - Benutzereingabe-Interface |
| 46 | - digitaler Eingang von 45, wobei n ganze Zahl grösser Null |
| 47 | - Verbindungsleitung |
| 48 | - Steuer- und Schalteinheit |
| 49 | - Spannungsüberwachung |
| 50 | - Verbindungsleitung |
| 51 | - Analog-Digital-Anschluss |
| 52 | - Energieversorgung |
| 53 | - Stromausgang |
| 100, 100a | - Sicherheitssystem |
| 200, 200a | - Sensorik |
| A - | Achse |
| AS - | Alarmsignal |
| BE - | Bewegungserkennung |
| DM - | Distanzmessung |
| DmS - | Distanzmessungs-Signal |
| EB - | Erfassungsbreite |
| G - | Gehäuse |
| H - | Höhe |
| HEB - | horizontaler Erfassungsbereich |
| HS - | Hinweissignal |
| MH - | Montagehöhe |
| NRw_{BE} - | Bewegungserkennungs-Nennreichweite |
| NRw_{DM} - | Distanzmessungs-Nennreichweite |
| Öw₁, Öw₂ - | Öffnungswinkel |
| SS - | Steuer- bzw. Schaltsignal |
| ssA - | sensorseitiger Alarm |
| tsA - | telefonseitiger Alarm |
| VEB - | vertikaler Erfassungsbereich |
| W - | Wand |
| ZI - | Zeitintervall |

## Patentansprüche

1. Sicherheitssystem (100, 100a) zur Bewegungserkennung (BE) in mindestens einem Raum, wobei die Bewegung mittels mindestens eines Distanzsensors (2, 2a) messbar ist und wobei in einem Mikrocontroller (15) ein Distanz-Erst-Istwert des Distanzsensors (2, 2a) mit einem Distanz-Zweit-Istwert des Distanzsensors (2, 2a) abgleichbar ist und ein Distanzmessungs-Signal (DmS) erzeugbar ist, mit dem mindestens ein Timer (16) triggerbar ist und nach Ablauf eines Zeitintervalls (ZI) ein systeminternes sensorseitiges Alarmsignal (ssA) ausgebbar ist.

2. Sicherheitssystem (100, 100a) nach Anspruch 1, **dadurch gekennzeichnet, dass** in den Mikrocontroller (15) ein Schwellenwert für den absoluten Wert des Distanzmessungs-Signals (DmS) eingebbar ist, der Mikrocontroller (15) einen Abgleich zwischen dem Schwellenwert und dem absoluten Wert des Distanzmessungs-Signals (DmS) durchführt und beim Überschreiten des Schwellenwertes den Timer (16) triggert.

3. Sicherheitssystem (100, 100a) nach Anspruch 1 oder 2, **dadurch gekennzeichnet, dass** das Sicherheitssystem (100, 100a) ein Notruf-Telefon (28) umfasst und dass das systeminterne sensorseitige Alarmsignal (ssA) ein telefonseitiges Alarmsignal (tsA) des Notruf-Telefons (28) triggert.

4. Sicherheitssystem (100, 100a) nach einem der vorhergehenden Ansprüche, **dadurch gekennzeichnet, dass** der Mikrocontroller (15) den durch den Eingang eines ersten Distanzmessungs-Signals (DmS) zum Ablauf des Zeitintervalls (ZI) getriggerten Timer (16) bei Eingang eines zweiten Distanzmessungs-Signals (DmS) resetet.

5. Sicherheitssystem (100, 100a) nach einem der vorhergehenden Ansprüche, **dadurch gekennzeichnet, dass** das Sicherheitssystem (100, 100a) mindestens einen Bewegungsmelder (1, 1a) umfasst, der bei einer Bewegungserkennung (BE) ein Signal an den Mikrocontroller (15) abgibt und dadurch die Stromversorgung des Distanzsensors (2, 2a) einschaltbar ist.

6. Sicherheitssystem (100, 100a) nach einem der vorhergehenden Ansprüche, **dadurch gekennzeichnet, dass** das Sicherheitssystem (100, 100a) einen Schalter zum Einstellen unterschiedlicher Zeitintervalle (ZI) des Timers (16) umfasst.

7. Sicherheitssystem (100, 100a) nach einem der vorhergehenden Ansprüche, **dadurch gekennzeichnet, dass** das Sicherheitssystem (100, 100a) eine Fernbedienung umfasst.

8. Sicherheitssystem (100, 100a) nach einem der vorhergehenden Ansprüche, **dadurch gekennzeichnet, dass** das Sicherheitssystem (100, 100a) ein Kommunikationsmodul (36) für externe Kommunikation in Form eines GSM-, KNX- oder TCPIP-Interfaces umfasst.

9. Sicherheitssystem (100, 100a) nach einem der vorhergehenden Ansprüche, **dadurch gekennzeichnet, dass** das Sicherheitssystem (100, 100a) in einen Konfigurationsmodus schaltbar ist und eine Timeout-Schaltung umfasst, die den Konfigurationsmodus automatisch abschaltet.

10. Sicherheitssystem (100, 100a) nach einem der vorhergehenden Ansprüche, **dadurch gekennzeichnet, dass** das Sicherheitssystem (100, 100a) in einen Abwesenheits- bzw. Ferienmodus schaltbar ist, der durch eine Bewegungserkennung (BE) durch den Bewegungsmelder (1, 1a) oder durch den Distanzsensor (2, 2a) automatisch abschaltbar ist und zeitverzögert wieder aktivierbar ist.

11. Sicherheitssystem (100, 100a) nach einem der vorhergehenden Ansprüche, **dadurch gekennzeichnet, dass** der Mikrocontroller (15) eine Testfunktion mit einem Zeitraffer umfasst.

12. Sicherheitssystem (100, 100a) nach einem der vorhergehenden Ansprüche, **dadurch gekennzeichnet, dass** der Timer (16) periodisch den Ablauf eines Selbsttests des Sicherheitssystems (100, 100a) triggert und die Daten des Selbsttests in einem Fehlerspeicher speicherbar sind.

13. Sicherheitssystem (100, 100a) nach Anspruch 12, **dadurch gekennzeichnet, dass** der Mikrocontroller (15) bei einem fehlerhaften Selbsttest das sensorseitige Alarmsignal (ssA) triggert und dieses ein telefonseitiges Alarmsignal mit einer Kennung auslöst.

14. Sicherheitssystem (100, 100a) nach einem der vorhergehenden Ansprüche, **dadurch gekennzeichnet, dass** das Sicherheitssystem (100, 100a) ein Funkmodul (26) umfasst, das auf das Senden einer individuellen Frequenz einstellbar ist und/oder zu den gesendeten Signalen eine Kennung mitsendet.

15. Sicherheitssystem (100, 100a) nach Anspruch 14, **dadurch gekennzeichnet, dass** das Sicherheitssystem (100, 100a) in eine Einlern-Funktion schaltbar ist und das Funkmodul (26) in Impulsen sendet.

16. Sicherheitssystem (100, 100a) nach einem der vorhergehenden Ansprüche, **dadurch gekennzeichnet, dass** das Sicherheitssystem (100, 100a) eine Spannungsüberwachung (49) in Form eines Spannungsreglers und/oder eines Spannungsdetektors umfasst.

17. Sicherheitssystem (100, 100a) nach Anspruch 16, **dadurch gekennzeichnet, dass** eine Meldung der Spannungsüberwachung (49) stundbar ist.

18. Sicherheitssystem (100, 100a) nach einem der vorhergehenden Ansprüche, **dadurch gekennzeichnet, dass** das Sicherheitssystem (100, 100a) einen Schalter umfasst, mit dem jeweils eine Voralarm-Vorstufe des sensorseitigen Alarmsignals (ssA) und eine Voralarm-Vorstufe des telefonseitigen Alarmsignals (tsA) quittier- und abschaltbär ist.

19. Sicherheitssystem (100, 100a) nach einem der vorhergehenden Ansprüche, **dadurch gekennzeichnet, dass** das Sicherheitssystem (100, 100a) eine Kamera und oder einen Scanner zum Erfassen des Belegungsbildes einer Tablettenschachtel umfasst.

20. Verfahren zur Anwendung eines Sicherheitssystems (100, 100a) nach einem der vorhergehenden Ansprüche 1-, **dadurch gekennzeichnet, dass** folgende Verfahrensschritte ausgeführt werden:
a) - Erfassen einer Bewegung (BE) mittels eines Bewegungsmelders (1, 1a);
b) - Einschalten eines Distanzsensors (2, 2a) bei einer erfassten Bewegung (BE);
c) - Durchführen einer Distanzmessung (DM) zu dem bewegten Objekt oder bewegten Person und Weiterleiten eines Distanzmessungs-Signals (DmS) an einen Mikrocontroller (15);
d) - Abgleichen des Distanzmessungs-Signals (DmS) mit einem vorgegebenen Schwellenwert;
e) - Einschalten eines Timers (16) bei Überschreiten des Schwellenwerts;
f) - Absetzen eines sensorseitigen Alarms (ssA) bei Verstreichen eines vorgegebenen Zeitintervalls (ZI) des Timers (16);
g) Auslösen eines telefonseitigen Alarms (tsA) durch Einschalten eines Notruf-Telefons (28).
